(19) Europäisches Patentamt
European Patent Office
Office européen des brevets

(11) Veröffentlichungsnummer: **0 457 183 B1**

# (12) EUROPÄISCHE PATENTSCHRIFT

(45) Veröffentlichungstag der Patentschrift: **16.08.95**

(51) Int. Cl.$^{6}$: **G01N 33/49**, G01N 33/52, B01D 39/20

(21) Anmeldenummer: **91107497.9**

(22) Anmeldetag: **08.05.91**

## (54) Vorrichtung und deren Verwendung zur Abtrennung von Plasma aus Vollblut.

(30) Priorität: **15.05.90 DE 4015589**

(43) Veröffentlichungstag der Anmeldung:
**21.11.91 Patentblatt 91/47**

(45) Bekanntmachung des Hinweises auf die Patenterteilung:
**16.08.95 Patentblatt 95/33**

(84) Benannte Vertragsstaaten:
**AT BE CH DE DK ES FR GB GR IT LI LU NL SE**

(56) Entgegenhaltungen:
**EP-A- 0 239 002**
**EP-A- 0 269 240**
**EP-A- 0 305 803**
**EP-A- 0 325 413**
**EP-A- 0 353 570**

(73) Patentinhaber: **BOEHRINGER MANNHEIM GMBH**

**D-68298 Mannheim (DE)**

(72) Erfinder: **Wilk, Hans-Erich, Dr.**
**Theodor Heuss-Strasse 2**
**W-6141 Einhausen (DE)**
Erfinder: **Vogel, Peter, Dr.**
**Schubertweg 5**
**W-6944 Hemsbach (DE)**
Erfinder: **Lerch, Rolf**
**Kanzelbachstrasse 22**
**W-6804 Ilvesheim (DE)**
Erfinder: **Schneider, Erich, Dr.**
**Märker Querschlag 6**
**W-6800 Mannheim 31 (DE)**
Erfinder: **Marschall, Andreas, Dr.**
**Levkojenweg 18**
**W-6800 Mannheim 31 (DE)**

EP 0 457 183 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung und deren Verwendung zur Abtrennung von Plasma aus Vollblut mit einer faserhaltigen Schicht, die eine Erythrozyten aggregierende Substanz enthält. Weiter betrifft die Erfindung ein Verfahren zur Abtrennung von Plasma aus Vollblut, bei dem Vollblut mit einer Erythrozyten aggregierenden Substanz kontaktiert und Erythrozyten durch eine faserhaltige Schicht zurückgehalten werden. Außerdem betrifft die Erfindung einen Testträger zur Bestimmung eines Blutbestandteils enthaltend einen Plasmagewinnungs- und einen Nachweisbereich. Schließlich betrifft die Erfindung die Verwendung von mit Polyvinylalkohol oder Polyvinylalkohol/Polyvinylacetat überzogenen Glasfasern.

Bei klinisch-chemischen Untersuchungen von Vollblut, insbesondere bei kolorimetrischen Tests, stören Erythrozyten oft wegen ihrer Eigenfarbe. Insofern sind Plasma oder Serum, die aus Vollblut gewonnen werden, das für Analysen auf gelöste Blutbestandteile bedeutendste Probenmaterial. Die gebräuchlichste Form der Abtrennung von Serum oder Plasma aus Vollblut ist die Zentrifugation. Diese ist jedoch insbesondere bei der Verwendung kleiner Probenmengen problematisch, auch ist die Trennung von Überstand und sogenanntem Blutkuchen, der sich bei der Zentrifugation absetzt, nicht immer einfach.

Von besonderer Problematik ist die Verwendung von Vollblut bei Schnelldiagnostika. Schnelldiagnostika bestehen aus reagentienhaltigen saugfähigen oder quellbaren Materialien, die als solches, z.B. als reagentiengetränktes Papier oder auf einem festen Träger befindlich mit der zu untersuchenden Flüssigkeit in Kontakt gebracht werden, wobei dann aufgrund der überwiegend sehr kurzen Reaktion eine meßbare Veränderung des ursprünglichen Schnelldiagnostikums z.B. vorzugsweise eine Farbänderung eintritt, die entweder visuell bewertet oder remissionsphotometrisch ausgemessen werden kann. Trübe und gefärbte Lösungen wie Blut können die Ablesung erheblich stören. Es hat daher nicht an Versuchen gefehlt, Vorrichtungen zur Verfügung zu stellen und Verfahren zu entwicklen, die zur Abtrennung von Plasma aus Vollblut, insbesondere auch für Schnelldiagnostika einsetzbar sind.

In EP-B-0 057 110 wird u. a. eine Vorrichtung zur Analyse von Vollblut beschrieben. Hierbei wird ein auf im wesentlichen nicht benetzendem Material immobilisiertes, die roten Blutkörperchen agglutinierendes Agenz eingesetzt. Bevorzugt werden als Träger Kügelchen verwendet. Bevorzugtes Material hierfür ist Polyacrylamid.

In US-A-3,146,163 und DE-A-14 98 577 wird zur Abtrennung von Plasma Vollblut auf ein mit einem Hämagglutinin, wie Phytohämagglutinin beschichtetes Material gegeben. Als mögliche Trägermaterialien werden Kunststoffe und faserige Materialien, wie Pappe, genannt.

In DE-A-34 41 149 wird ein Verfahren zur Vollblutauftrennung beschrieben, das darin besteht, daß Vollblut auf eine mit Lectin imprägnierte Matrix aufgegeben wird und Plasma oder Serum hierbei abgetrennt werden. Die Matrix besteht aus saugfähigem Material mit einem relativen Strömungswiderstand von bis zu 40 %. Es kommen hierfür Materialien mit Gewebe- oder Faserstruktur in Frage, die einen möglichst geringen Strömungswiderstand besitzen. Als bevorzugte Materialien werden Baumwoll- und Viskosegewebe und Zellulosematerialien genannt. Ein offensichtlicher Nachteil des Verfahrens ist jedoch, daß das abgetrennte Plasma oder Serum mit einem Verdünnungsmittel aus der Matrix herausgespült werden muß.

Eine weitere Vorrichtung zur Abtrennung von Plasma oder Serum aus Vollblut ist in EP-A-0 295 526 beschrieben. Die Vorrichtung enthält eine saugfähige Matrix, die mit Blutzellen agglutinierendem Agenz behandelt ist. Als Blutzellen agglutinierendes Agenz werden Thrombin oder Lectin genannt. Für die saugfähige Matrix werden hydrophile Pulver, schwammähnliche und tonige Materialien, Gewebe und Polymere empfohlen. Unter Polymeren werden auch faserhaltige Papiere verstanden. Bevorzugte Matrix ist Filterpapier.

EP-A-0 194 502 hat als Gegenstand ein Mittel zur Abtrennung von Plasma oder Serum aus Vollblut, bei dem als Abtrennschicht eine mit Lectin imprägnierte saugfähige Matrix verwendet wird. Die Matrix besteht vorzugsweise aus Textilgewebe oder einem zellulosehaltigen Material.

In EP-A-0 325 413 sind Polykationen an ein saugfähiges, festes Material immobilisiert, so daß eine kationische Oberfläche entsteht, an die bei Kontakt mit Vollblut Erythrozyten gebunden werden. Bevorzugtes Trägermaterial ist Papier.

In EP-A-0 133 895 wird zur Plasmaabtrennung aus Vollblut ein mit Substanzen mit mehreren polaren Gruppen imprägniertes saugfähiges, poröses Material eingesetzt. Als solches sind u. a. allgemein genannt Papiere, Vliese und Gewebe.

In EP-A-0 305 803 ist eine Vorrichtung zur Abtrennung von Blutzellen aus erythrozytenhaltigen Körperflüssigkeiten beschrieben, die aus einer faserhaltigen Filterschicht besteht, die erythrozytenagglutinierende Antikörper und gegebenenfalls ein Lectin enthält oder aus einer Filterschicht aus Glasfasern und einem Lectin besteht.

EP-A-0 269 240 hat eine Vorrichtung zur Abtrennung von Plasma von roten Blutkörperchen zum Gegenstand, bei der Vollblut auf einen Filter trifft, der aus Glasfasern besteht, die ein erythrozytenagglutinierendes Agenz tragen können.

Von den aus dem Stand der Technik bekannten Methoden zur Vollblutauftrennung haben sich insbesondere diejenigen für die Praxis als interessant erwiesen, bei denen Vollblut mit einer erythrozytenaggregierenden Substanz kontaktiert und Erythrozyten durch eine faserhaltige Schicht zurückgehalten werden. Nachteile solcher Erythrozytenabtrennschichten bestehen jedoch häufig darin, daß sie die Erythrozyten nur bei Einsatz verdünnter Proben zufriedenstellend abtrennen und/oder daß bei Kontakt der abzutrennenden Erythrozyten mit der Abtrennschicht Hämolyse eintritt. Wenn vor Abtrennung der Erythrozyten eine Vollblutprobe erst verdünnt werden muß, erfordert dies einen zusätzlichen Schritt, der umständlich ist und anschließend durchgeführte Meßergebnisse verfälschen kann. Ein Verdünnungsschritt ist deshalb in der Praxis unerwünscht.

Hämolyse führt zur Freisetzung von Hämoglobin aus den Erythrozyten und damit zur Verfärbung des Plasmas oder Serums. Solche Verfärbungen können kolorimetrische Tests erheblich stören. Darüber hinaus werden bei der Hämolyse auch andere Erythrozyteninhaltsstoffe in das Plasma freigesetzt. Auch wenn die resultierende hämoglobinbedingte Verfärbung nicht zu einer Störung eines kolorimetrischen Testes führen würde, kann aber die Bestimmung bestimmter Parameter auch bei geringer Hämolyse bereits erheblich verfälscht werden. Beispielsweise werden bei der Hämolyse von Erythrozyten Kaliumionen freigesetzt, so daß eine Bestimmung von Kalium in Plasma dadurch völlig verfälscht werden kann. So sind z. B. aus EP-A-0 045 476 Mittel und Verfahren zur Abtrennung von Plasma oder Serum aus Vollblut mit Glasfaserschichten bekannt, die für eine gute Erythrozytenabtrennung aus unverdünntem Vollblut einsetzbar sind. Die durch Glas verursachte geringe Hämolyse von Erythrozyten genügt jedoch, um eine Kaliumbestimmung aus Vollblut zu verfälschen.

Aufgabe der vorliegenden Erfindung war es deshalb, eine Möglichkeit zu finden, aus unverdünntem Blut die zellulären Bestandteile, insbesondere Erythrozyten, von Plasma gut abzutrennen und bei dieser Abtrennung wenig Haemolyse zu verursachen. Die Abtrennung sollte insbesondere auch auf Schnelldiagnostica möglich sein.

Diese Aufgabe wird durch die Erfindung gelöst, wie sie in den Patentansprüchen charakterisiert ist.

Gegenstand der Erfindung ist eine Vorrichtung zur Abtrennung von Plasma aus Vollblut mit einer glasfaserhaltigen Schicht, die eine Erythrozyten aggregierende Substanz enthält und wobei die Glasfasern dieser Schicht mit Polyvinylalkohol oder Polyvinylalkohol/Polyvinylacetat überzogen sind.

Weiter ist Gegenstand der Erfindung ein Verfahren zur Abtrennung von Plasma aus Vollblut, bei dem Vollblut mit einer Erythrozyten aggregierenden Substanz in Kontakt gebracht wird und beim Durchtritt durch eine glasfaserhaltige Schicht, in der die Glasfasern mit Polyvinylalkohol oder Polyvinylalkohol/Polyvinylacetat überzogen sind, Erythrozyten dort zurückgehalten werden.

Außerdem ist Gegenstand der Erfindung ein Testträger zur Bestimmung eines Blutbestandteils enthaltend einen Plasmagewinnungs- und einen Nachweisbereich dadurch gekennzeichnet, daß der Plasmagewinnungsbereich eine Vorrichtung enthält, die eine glasfaserhaltige Schicht, die eine Erythrozyten aggregierende Substanz trägt, beinhaltet, wobei die Glasfasern dieser Schicht mit Polyvinylalkohol oder Polyvinylalkohol/Polyvinylacetat überzogen sind.

Schließlich ist Gegenstand der Erfindung die Verwendung von mit Polyvinylalkohol oder Polyvinylalkohol/Polyvinylacetat beschichteten Glasfasern, die eine Substanz tragen, die zur Agglomeration von Erythrozyten führt, zur Abtrennung von Plasma aus Vollblut.

Überraschenderweise wurde erfindungsgemäß gefunden, daß eine Glasfaserschicht in der die Glasfasern mit Polyvinylakohol oder Polyvinylakohol/Polyvinylacetat überzogen sind und eine Substanz tragen, die zur Agglomeration von Erythrozyten führt, dazu geeignet ist, aus unverdünntem Blut die zellulären Bestandteile von Plasma sehr gut abzutrennen, ohne daß hierbei eine wesentliche Hämolyse eintritt.

Aus EP-A-0 239 002 sind zwar mit Polyvinylalkohol oder Polyvinylalkohol/Polyvinylacetat überzogene Glasfasern und solche Fasern enthaltende Schichten bekannt. Dort dient ein solcher Überzug aber lediglich dazu, Fasern gerinnungsneutral zu machen. Daß ein Überzug von Polyvinylalkohol/Polyvinylacetat einen Hämolyse unterdrückenden Einfluß haben könnte, ist dieser Schrift nicht zu entnehmen. Im übrigen werden keine Erythrozyten aggregierende Substanzen erwähnt.

Ebenso ist in EP-A-0 353 570 eine mit Reagenz ablösbar imprägnierte Trägermatrix beschrieben, die aus Polyvinylalkohol beschichtetem Glas besteht. Die Trägermatrix wird nicht zur Plasmaabtrennung aus Blut eingesetzt. Daß beschichtetes Glas Erythrozyten aggregierende Substanzen tragen kann, wird in dieser Schrift nicht gelehrt. Ebenso findet der Aspekt der Hämolyse keine Berücksichtigung.

Für die vorliegende Erfindung können Glasfasern unterschiedlichen Durchmessers verwendet werden. Das Glasmaterial kann aus alkalihaltigem oder alkalifreiem Borosilikatglas, aber auch aus reinem Quarzglas bestehen. Fasermaterial aus anderen technischen Gläsern, z.B. borfreien Alkaligläsern, Kristallglas, Bleiglas und anderen befinden sich in der notwendigen Abmessung der Faser nicht im Handel und konnten daher nicht untersucht werden. Es wird aber davon ausgegangen, daß auch sie geeignet sind. Der mittlere Durchmesser der Glasfasern kann zwischen 0,5 µm und 2,5 µm, insbesondere zwischen 0,5 µm und 1,5 µm liegen. Die Faserdurchmesser können entsprechend der Herstellung stark streuen, sollten jedoch eine Obergrenze von ca. 10 µm nur ausnahmsweise überschreiten. Ihre Länge ist nur durch die Art der Stapelung begrenzt, hat aber ansonsten keinen Einfluß. Je nach Art der Stapelung werden Dichten von 0,1-0,5, überlicherweise 0,2-0,4 g/cm$^3$ als vorteilhaft gefunden. Die Glasfasern können lose gestapelt sowie in Form von Papieren, Vliesen oder Filzen aber auch gehalten durch eine äußere Form in jeder gewünschten Gestalt Verwendung finden.

Polyvinylakohol wird überlicherweise aus Polyvinylacetat durch Verseifung hergestellt, wobei je nach den gewünschten Eigenschaften des Produktes eine vollständige oder teilweise Verseifung durchgeführt wird. Für den erfindungsgemäßen Zweck ist sowohl ein voll- als auch ein teilverseiftes Produkt verwendbar. Polyvinylalkohole, die im Handel in großer Menge angeboten werden, unterscheiden sich insbesondere durch ihr durchschnittliches Molekulargewicht, welches normalerweise zwischen etwa 10.000 und 100.000 liegt und in einigen Sonderfällen auch noch wesentlich höhere Werte erreichen kann, sowie durch den Restgehalt an Acetylgruppen. Die niedermolekularen Verbindungen, die ca. 5-15 %, insbesondere etwa 10 % Acetylgruppen enthalten, sind in Wasser am leichtesten löslich, hochmolekulare und/oder stärker acetylhaltige Produkte sind dagegen in Wasser weniger löslich. Von Einfluß auf die Löslichkeit ist ferner die Wechselwirkung der Polyvinylakoholketten untereinander. Durch eine parallele Lagerung der Polymerketten in bestimmten Bereichen entstehen "kristalline" Bezirke, wobei die Orientierungstendenz um so größer ist, je regelmäßiger die Ketten aufgebaut sind und je geringer der Anteil der Acetylgruppen ist, die einer Orientierung am stärksten entgegenwirken. Bei einem Verseifungsgrad von 97-100 mol%, d.h. bei einem Acetylierungsgrad von 3-0 mol% nimmt deshalb die "Kristallinität" besonders stark zu, wodurch umgekehrt die Kaltwasserlöslichkeit stark abnimmt.

Die Wasserlöslichkeit kann weiterhin durch Nachbehandlung mit Aldehyden (Acetalisierung) oder andere chemische Umwandlung der Alkoholgruppen verringert werden. Erfindungsgemäß brauchbar sind insbesondere solche Polyvinylakohole mit einer sehr geringen Kaltwasserlöslichkeit. Die Produkte sollen bei 20°C in Wasser nur langsam oder überhaupt nicht gelöst werden. Bei Temperaturen von 50-100°C, insbesondere bei Temperaturen von über 60°C ist eine Löslichkeit in Wasser aber nicht nachteilig.

Erfindungsgemäß sind die zur Abtrennung von Plasma aus Vollblut eingesetzten Glasfasern so mit einer Polyvinylakohol oder Polyvinylakohol/Polyvinylacetat-Schicht bedeckt, daß die gesamte Glasoberfläche belegt ist. Hierzu reichen bereits relativ kleine Mengen, insbesondere etwa 0,5-20 Gew.-%, vorzugsweise 1-10 Gew.-% Polyvinylalkohol oder Polyvinylakohol/Polyvinylacetat bezogen auf die Glasfasern.

Zur erfindungsgemäßen Belegung Polyvinylakohol- oder Polyvinylakohol/Polyvinylacetat überzogener Glasfasern mit Erythrozyten aggregierenden Substanzen eignen sich grundsätzlich alle Substanzen, die in der Lage sind, in Konzentrationen, die nicht zu merklicher Hämolyse führen, Erythrozyten unabhängig von Blutgruppen so zu größeren Verbänden zusammenzulagern, daß sie durch die Faserschicht aus Blut abgetrennt werden können. Bevorzugt werden hierzu kationische Polymere, Lectine, Antikörper gegen Erythrozyten oder Mischungen einzelner oder aller dieser Substanzen eingesetzt.

Als kationisches Polymer eignen sich grundsätzlich alle organischen oder anorganischen Polymere, die mehr als 10 positive Ladungen pro Molekül, vorzugsweise mehr als 20, aufweisen. Als besonders bevorzugt haben sich Copolymere aus N,N,N',N'-Tetramethyl-1,6-hexandiamin und 1,3-Dibrompropan (Polybren®), Polyethylenimin (Polymin®) und Polypiperidiniumsalz, beispielsweise Polydimethylpiperidiniumchlorid erwiesen. Ganz besonders bevorzugt ist Polybren®, insbesondere solches eines Molekulargewichts von weniger als 6000.

Als Lectine sind vor allem solche einsetzbar, die an alle menschlichen Erythrozyten zu binden vermögen und diese so agglutinieren, d. h. sie sollten nicht blutgruppenspezifisch sein. Bevorzugt werden die Lectine von Solanum tuberosum (Kartoffel), Lycopesicon esculantum (Tomate), Ricinus communis oder von Phytolacca americana (Kermesbeere) jeweils allein, als Kombination von mehreren oder allen genannten eingesetzt.

Antikörper, die erfindungsgemäß eingesetzt werden können, binden an Oberflächenstrukturen von Erythrozyten. Vorzugsweise sollten die Antikörper blutgruppenunspezifisch sein, ansonsten müßten die Antikörper der vorliegenden Blutprobe angepaßt sein. Es sind monoklonale oder polyklonale Antikörper verwendbar, wobei häufig der Einsatz "einfacher" herzustellender polyklonaler Antikörper ausreicht.

Erfindungsgemäß hat sich als Vorrichtung zur Abtrennung von Plasma aus Vollblut insbesondere Glasfaservlies als vorteilhaft erwiesen. Die Herstellung eines erfindungsgemäßen Glasfaservlieses kann dadurch erfolgen, daß ein entsprechendes Glasfaservlies nachträglich mit einer Lösung von Polyvinylalkohol in Wasser oder einem geeigneten organischen Lösungsmittel behandelt und anschließend getrocknet wird, vorzugsweise bei Temperaturen von über 60°C, ganz besonders bei 90-140°C, oder indem man bei der Herstellung des Glasfaservlieses Polyvinylakohol oder Polyvinylakohol/ Polyvinylacetat zusetzt. Glasfaservliese werden so hergestellt, daß man die trockenen und verfilzten Glasfasern, die einen mittleren Durchmesser von 0,5-2,5 µm, insbesondere zwischen 0,5 und 1,5µm aufweisen, in einem sehr großen Überschuß an Wasser suspendiert, dadurch vereinzelt und diese "Bütte" analog den in der Papierherstellung üblichen Verfahren und mit Hilfe der dort üblichen Maschinen zu dünnen Schichten formt und trocknet. Der Bütte zugesetzte Polyvinylalkoholpulver oder -fasern verteilen sich bei der Aufschlemmung der Glasfasern gleichmäßig in der Masse und werden beim anschließenden Herstellen des Vlieses soweit gelöst bzw. aufgeschmolzen, daß sie anschließend an die Trocknung des Vlieses einen gleichmäßigen Überzug auf den Glasfasern bilden.

Zum Aufbringen Erythrozyten aggregierender Substanz auf eine glasfaserhaltige Schicht, wird letztere vorzugsweise mit einer Lösung der entsprechenden Substanz getränkt, wobei z.B. die Tränklösung auf die Glasfaserschicht aufgegeben oder diese in die Tränklösung eingetaucht wird.

Ein Glasfaservlies, dessen Glasfasern mit Polyvinylalkohol oder Polyvinylalkohol/Polyvinylacetat überzogen sind, weist eine gegenüber unbehandeltem Glasfaservlies gesteigerte Naßfestigkeit auf. Während unbehandelte Glasfaservliese, insbesondere in technisch und für eine größere Produktion erforderlicher Menge und Größe wegen ihrer geringen Naßfestigkeit nicht in Tränklösungen eingetaucht und mit Reagentien imprägniert werden können ohne zu reißen, ist dies mit Polyvinylakohol oder Polyvinylakohol/ Polyvinylacetat beschichteten Glasfaservliesen gut möglich. Um eine möglichst homogene Imprägnierung mit Polyvinylakohol oder Polyvinylalkohol/Polyvinylacetat beschichteten Glasfaservliesen mit Erythrozyten aggregierender Substanz zu erreichen, können deshalb vorteilhafterweise so vorbehandelte Glasfaservliese durch Eintauchung in eine Lösung der aufzubringenden Substanz imprägniert werden.

Als Lösungsmittel kann jede Flüssigkeit gewählt werden, die die Erythrozyten zusammenballende Substanz ausreichend gut löst, die die Eigenschaften dieser Substanz nicht negativ beeinflußt und die nach Imprägnierung des mit Polyvinylalkohol oder Polyvinylalkohol/Polyvinylacetat überzogenen Glasfaservlieses mit der Erythrozyten aggregierenden Substanz wieder so entfernt werden kann, daß die Plasmaabtrenneigenschaften des so hergestellten Filtermaterials nicht beeinträchtigt werden. In der Mehrzahl der Fälle wird Wasser bzw. wässrige Pufferlösung das Lösungsmittel der Wahl sein.

Nach dem Imprägniervorgang schließt sich eine Trocknungsstufe an, wenn das Lösungsmittel der Erythrozyten aggregierenden Substanz gesondert entfernt werden muß. Im Falle von Wasser als Lösungsmittel ist dies regelmäßig der Fall. Es muß hierbei je nach der Erythrozyten aggregierenden Substanz entschieden werden, bei welcher Temperatur der Trocknungsvorgang durchgeführt werden und wie lange er dauern soll. In der Regel werden die Temperaturen zwischen 40 und 90 °C betragen und die Trocknungsdauer zwischen 1 und 10 Minuten liegen.

Die Konzentration der Erythrozyten aggregierenden Substanz auf der Oberfläche von mit Polyvinylalkohol oder Polyvinylalkohol/ Polyvinylacetat behandelten Glasfaserschichten ist für den erfindungsgemäßen Einsatz abhängig von der Art der jeweils eingesetzen aggregierend wirkenden Substanz und der Menge an Blut, von dem Plasma abgetrennt werden soll. Sie muß auf jeden Fall so groß sein, daß vollständige Plasmaabtrennung eintritt, sie darf jedoch nicht so hoch sein, daß störende Haemolyse auftritt. Dies kann durch einfache Versuche ermittelt werden, indem auf mit verschiedenen Konzentrationen an Erythrozyten aggregierenden Substanzen imprägnierte Glasfaserschichten Blut aufgegeben wird und das durch Schwerkraft und/oder Kapillarkraft durch diese Schicht gelangte Plasma daraufhin untersucht wird, ob es Erythrozyten oder Hämoglobin enthält. Solche Versuche können analog Beispiel 2 durchgeführt werden. Zur Charakterisierung der eingesetzten Konzentration an kationischen Polymeren hat sich die Zahl positiver Ladungen pro Gramm beschichteter Glasfaserschicht als brauchbar erwiesen. Sie beträgt erfindungsgemäß $10^{18}$ bis $10^{21}$ Ladungen pro Gramm, vorteilhafterweise $5 \cdot 10^{19}$ bis $10^{21}$ Ladungen pro Gramm.

Eine erfindungsgemäße Vorrichtung zur Abtrennung von Plasma aus Vollblut kann aus mit Polyvinylalkohol oder Polyvinylalkohol/Polyvinylacetat überzogenen ein kationisches Polymer tragenden Glasfasern bestehen die lose gestapelt sind oder die in Form von Papieren, Vliesen oder Filzen vorliegen. Sie können aber auch gehalten durch eine äußere Form in jeder gewünschten Gestalt Verwendung finden. Beispielsweise können mit entsprechend behandelten Glasfasern gefüllte Säulen, Filternutschen oder andere geeignete Gefäße dazu verwendet werden, um durch einfaches Durchlaufen von Blut Plasma ohne Zentrifugation zu gewinnen.

Insbesondere kann eine erfindungsgemäße Vorrichtung vor allem ein Glasfaservlies dessen Glasfasern mit Polyvinylalkohol oder Polyvinylalkohol/Polyvinylacetat überzogen sind und die eine Erythrozyten aggregierende Substanz tragen in einem Testträger zur Bestimmung eines Blutbestandteils mit einem Plasmagewinnungs- und einem Nachweisbereich im Plasmagewinnungsbereich eingesetzt werden. Beispiele für Ausgestaltungsmöglichkeiten solcher Testträger sind in Analogie zu den beispielsweise in der EP-A-0 045 476, EP-A-0 239 002 oder EP-A-0 133 895 beschriebenen Mitteln, auf die hiermit Bezug genommen wird, möglich.

In Fig. 1 ist als Beispiel eine Ausgestaltungsform eines Testträgers dargestellt, der eine erfindungsgemäße Plasmaabtrennschicht enthält.

Fig. 2 a und b zeigen Funktionskurven für die Korrelation von Remission [%R] und Analytenkonzentration [mmol / l] von Testträgern gemäß Fig. 1 mit unterschiedlichen Abtrennschichten für Blut und Plasma als Probeflüssigkeiten.

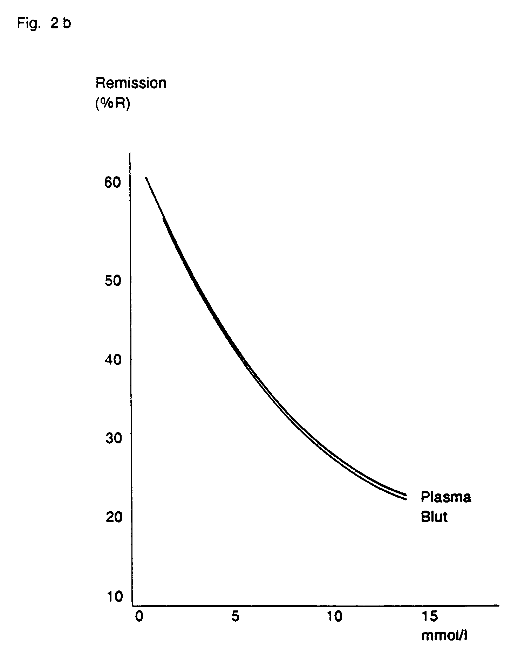

Für Fig. 2 a wurde ein Glasfaservlies mit 2 % Polyvinylalkohol gemäß EP-A-0 239 002 als Abtrennschicht verwendet.

Für Fig. 2 b wurde ein erfindungsgemäßes Glasfaservlies mit 2% Polyvinylalkohol und kationischem Polymer verwendet.

Fig. 1: Auf einer inerten Trägerfolie (5), beispielsweise einer Kunststoffolie, ist eine Transportschicht (2) befestigt, die zum Transport der Probenflüssigkeit aus der Probenaufgabe- und Plasmagewinnungszone (7) in den Nachweisbereich (8) dient. Als Transportschicht (2) ist prinzipiell jedes Material geeignet, das die zu untersuchende Flüssigkeit aus der Probenaufgabezone (7) in den Nachweisbereich (8) zu transportieren in der Lage ist und sie dabei nicht so verändert, daß die Analyse beeinträchtigt wird. Besonders vorteilhaft ist es, als Transportschicht (2) ein Glasfaservlies einzusetzen. Die Transportschicht (2) teilweise überdeckend ist eine erfindungsgemäße Plasmaabtrennschicht (3). Über der Abtrennschicht (3) ist eine Schutzschicht (4) angebracht, die verhindern soll, daß bei der Probenaufgabe, beispielsweise mittels einer Pipette, die Abtrennschicht (3) beschädigt wird. Bewährt hat sich hierfür ein Netz aus inertem Material, beispielsweise aus Kunststoff. Schutzschicht (4) und Abtrennschicht (3) sind auf der inerten Trägerfolie (5) befestigt. Dies kann beispielsweise mittels eines Schmelzklebestreifens (6) erfolgen. Seitlich von der Transportschicht (2) ist eine aus durchsichtigem Kunststoff bestehende Trägerfolie mit einer die für die Untersuchung des gewonnenen Plasmas notwendigen Reagenzien enthaltenden Schicht (1) befestigt. Vorteilhafterweise geschieht dies über eine Klebestelle (9), beispielsweise einen Schmelzkleberstreifen. Die Filmschicht (1) ist so angeordnet, daß sie beim Niederdrücken der durchsichtigen Trägerfolie in Richtung der inerten Trägerfolie (5) mit der Transportschicht (2) in einen einen Flüssigkeitsübergang ermöglichenden Kontakt gebracht werden kann.

Zur Durchführung der Bestimmung eines Analyts in Blut wird die Probe auf die Schutzschicht (4) gegeben. Das Blut dringt in die Abtrennschicht (3) ein, Erythrozyten werden aggregiert und zurückgehalten. Durch Kapillarkräfte wird das so gewonnene Plasma in die Nachweiszone (8) gesaugt. Durch Druck auf die Trägerfolie mit der Reagenzschicht (1) wird das Plasma in der Transportschicht (2) mit der Reagenzschicht in Kontakt gebracht, Flüssigkeit dringt in die Reagenzschicht ein und die Bestimmungsreaktion wird ausgelöst. Im Fall einer kolorimetrischen Reaktion wird die Reaktion beispielsweise anhand der Verfärbung in der Reagenzschicht durch die Trägerfolie der Filmschicht (1) hindurch visuell beobachtet oder reflexionsphotometrisch gemessen.

Die durch die Erfindung erreichten Vorteile sind im wesentlichen darin zu sehen, daß aus Vollblut ohne vorherige Verdünnung und ohne störende Hämolyse zelluläre Bestandteile entfernt werden können. Zusammen mit Erythrozyten können im wesentlichen auch alle übrigen korpuskulären Bestandteile des Blutes, wie Leukozyten und Thrombozyten abgetrennt werden.

Dadurch daß die erfindungsgemäße Plasmaabtrennschicht sehr effektiv ist, kann man auch aus kleinen Blutproben schnell Plasma isolieren.

Aufgrund fehlender störender Einflüsse der Plasmaabtrennschicht auf die kontaktierende Blutprobe wird bei anschließender Bestimmung von Plasmainhaltsstoffen keine Blut-/Plasmadifferenz festgestellt, d. h. aus Blut und Plasma werden übereinstimmende Werte erhalten.

In den folgenden Beispielen soll die Erfindung näher erläutert werden.

Beispiel 1

a) Glasfaservlies-Herstellung
Als Ausgansmaterialien dienen:
1.000 l entionisiertes Wasser
2 kg Glasfasern Typ 108 A (John Manville, USA)
0,04 kg Polyvinylalkohol, Typ Kuralon VPB 105-2 (Rohtex Textil GmbH, BRD)
Als Papiermaschine wird eine Schrägsiebmaschine (Voith, Heidenheim, BRD) verwendet. Die im Wasser suspendierten Fasern werden auf ein Schrägsieb gepumpt. Während die Flüssigkeit abfließt, bzw. durch Vakuum abgesaugt wird, orientieren sich die Fasern auf der Sieboberfläche und werden als Vlies über Trockenzylinder getrocknet. Die Trocknung findet bei 125°C statt, bis eine Endfeuchte von 0,5-1,5 Gew.-% erreicht ist. Die Absaug- und die Produktionsgeschwindigkeit werden mit 2 m/pro Minute so gewählt, daß ein Material eines Flächengewichts von 60 g/$m^2$ und einer Dicke von 0,45 mm entsteht.

b) Tränkung des Glasfaservlieses mit kationischem Polymer:

Das Glasfaservlies wird mit einer 0,7 %igen Lösung von Polybren® (BASF, Ludwigshafen, BRD) getränkt. Die spezifische Tränkaufnahme beträgt ca. 370 ml/$m^2$. Getrocknet wird das Glasfaservlies in einem Schwebetrockner bei 80°C bei einer Geschwindigkeit von einem 1 m/pro Minute.

c) Tränkung des Glasfaservlieses mit Antikörpern:

Das Glasfaservlies wird analog Beispiel 1 b) mit einer Lösung von Anti-Erythrozyten Antikörpern (Dakopatts) in physiologischer Kochsalzlösung (Konzentration 4 mg/ml) getränkt (Tränkaufnahme 370 ml/$m^2$). Getrocknet wird bei 60 °C in einem Schwebetrockner bei einer Geschwindigkeit von 1 Minute.

d) Tränkung des Glasfaservlieses mit Lectin

Das Glasfaservlies wird mit einer Lösung von Lectin aus Ricinus communis (RCA 120, Boehringer Mannheim GmbH, Mannheim, BRD) in Phosphat-gepufferter (1,25 mmol/l) physiologischer Kochsalzlösung (Konzentration 1 mg/ml) getränkt und analog Beispiel 1 c) getrocknet.

e) Tränkung des Glasfaservlieses mit Antikörpern und kationischem Polymer

Eine Lösung aus 0,7 % Polybren® und 1 mg/ml Anti-Erythrozyten Antikörpern (Dakopatts) in 10 mM Hepes-Puffer, pH 7,2, wird analog Beispiel 1c) in das Glasfaservlies getränkt und getrocknet.

## Beispiel 2

Plasmaabtrenneigenschaften

Die Abtrennleistung der in Beispiel 1 hergestellten erfindungsgemäßen Glasfaservliese wird in einem Aufbau gemessen, bei dem auf ein zum Plasmatransport dienendes Glasfaservlies mit 25 g/$m^2$ Flächengewicht (Binzer, BRD) und der Maße 20 x 6 mm an einem Ende ein Abtrennvlies der Maße 6 x 6 mm vollfächig aufgelegt ist.

Auf das Abtrennvlies werden 32 µl Blut eines Haematokrits von 48 % aufpipettiert und die Erythrozytenfront auf dem Transportvlies nach 2 Minuten gemessen.

Außerdem wird die Haemolyse gemessen, die 2,2 mg Abtrennvlies mit 1 dl Blut verursachen.

Erfindungsgemäße glasfaserhaltige Schichten werden mit Plasmaabtrennschichten des Standes der Technik verglichen. Die Ergebnisse sind aus Tabelle 1 ersichtlich. Nur die erfindungsgemäßen Plasmaabtrennschichten trennen Erythrozyten sehr gut ohne merkliche Hämolyse ab.

Tabelle 1

| Plasmaabtrennschicht | Erythrozytenfront [mm] | Haemolyse [g Hb/dl] |
|---|---|---|
| Glasfasern ohne Polyvinylalkohol und ohne kationisches Polymer | 1-2 | 0,45 |
| Glasfasern mit 2 % Polyvinylalkohol | 2,8 | 0,05 |
| Glasfasern mit Polybren® | <0,2 | 0,3 |
| Glasfasern mit 2 % Polyvinylalkohol und Polymin P | <0,2 | 0,1 |
| Glasfasern mit 2 % Polyvinylalkohol und Polybren® | <0,2 | 0,01 |
| Papier (Whatman 31 ET Chrom) mit Polybren® (gemäß EP-A-0 325 413) | keine Plasmaabtrennung | |
| Glasfasern mit 2 % Polyvinylalkohol und Lectin | <0,2 | 0,01 |
| Glasfasern mit 2 % Polyvinylalkohol und Antikörpern | <0,2 | 0,01 |
| Glasfasern mit 2 % Polyvinylalkohol, Antikörpern und Polybren® | <0,2 | keine Hämolyse meßbar |

Beispiel 3

Blut-/Plasmadifferenz bei Verwendung eines Testträgers zum Nachweis von Kalium in Blut und Plasma.

Es werden drei Plasmaabtrennschichten hergestellt und jeweils auf einem Testträger gemäß Fig. 1 als Schicht (3) eingesetzt.

Plasmaabtrennschichten sind:

a) Glasfasern mit 2 % Polyvinylalkohol (gemäß EP-A-0 239 002, hergestellt gemäß Beispiel 1 a)

b) Glasfasern mit 2 % Polyvinylalkohol und kationischem Polymer (hergestellt gemäß Beispiel 1 b)

Die Plasmaabtrennschichten werden jeweils als 6 x 6 mm große Vliese mit einem Schmelzkleberstreifen unter einem 6 x 6 mm großen Polyamid-Schutznetz und wie in Beispiel 2 beschrieben über einem 20 x 6 mm großen Glasfaservlies (25 g/m$^2$, Binzer, Bundesrepublik Deutschland) auf einer 150 mm langen und 6 mm breiten weißen Polyesterfolie als Trägerfolie befestigt.

Als Reagenzschicht für den Kaliumnachweis wird eine 15 mm lange und 6 mm breite durchsichtige Polyesterfolie (200 µm dick), die mit den zur Analytbestimmung notwendigen Reagenzien beschichtet ist, mit einem Schmelzkleberstreifen auf der weißen Polyesterfolie befestigt.

Zur Herstellung der Reagenzschicht wird eine Mischung der nachfolgenden Zusammensetzung mit einer Naßdicke von 300 µm auf die durchsichtige Polyesterfolie aufgebracht und getrocknet:

| | |
|---|---|
| Vinylacetat-Maleinsäuredibutylester-Copolymer (Mowilith® 35/73, Hoechst Ag, Frankfurt, BRD) | 14,7 g |
| 2,2-Diphenyl-1-cyano-acrylsäure-äthylhexylester (Uvinul® N539, BASF, Ludwigshafen, BRD) | 18,4 g |
| 4-(2,6-Dibrom-4-nitro-phenylazo-)-2-octadecyloxy-naphtol-1 (hergestellt gemäß Beispiel 3c) | 0,130 g |
| Valinomycin | 0,600 g |
| Diatomeenerde (Celatom® MW 25, Eagle-Picher, Cincinatti, USA) | 28,2 g |
| Essigsäurebutylester | 50,7 g |

Auf diese Schicht wird mit einer Naßfilmdicke von 150 µm eine zweite Schicht folgender Zusammensetzung aufgebracht und gleichfalls getrocknet.

| | |
|---|---|
| Hydroxyäthylcellulose (Natrosol® 250G, Hercules Inc., Willmington, Delaware, USA) in Wasser | 41,5 g |
| N,N-Bis-(hydroxyäthyl)-aminoäthansulfonsäure | 8,5 g |
| Ethanol | 64 ml |
| mit LiOH auf pH 7,8 eingestellt. | |

Diese Reagenzschicht wird nach Aufteilen in 15 x 6 mm große Stücke in der oben beschriebenen Weise mit dem Testträger verbunden.

c) 4-[(2,6-Dibrom-4-nitrophenyl)azo]-2-octadecyloxy-1-naphthol

ca) 2-Octadecyloxynaphthalin

Man gibt in einen 4 l-Dreihalskolben mit Rührer, Kühler und Thermometer 172,8 g (1,2 mol) 2-Naphthol (98 %) zu einer Lösung von 48 g (1,2 mol) Natriumhydroxid (99 %) in 1 l Ethanol, fügt nach erfolgter Lösung 417 g (1,25 mol) n-Octadecylbromid hinzu und erhitzt die Reaktionsmischung 14 Stunden unter Rückfluß. Nach Zugabe von weiteren 1 l Ethanol wird die heiße Lösung zur Entfernung anorganischen Materials über ein Seitzfilter abgesaugt und das schwach rosa gefärbte Filtrat durch 30minütiges Einstellen in ein Eisbad zur Kristallisation gebracht. Nach Absaugen der fast farblosen Kristalle wäscht man den Filterkuchen portionsweise mit ca. 700 ml Ethanol und erhält nach Trocknen über Diphosphorpentoxid 371,9 g (93,7 % der Theorie) 2-Octadecyloxynaphthalin, farblose Kristalle, Fp. 64-68 °c.

DC: Kieselgel 60 (Merck), Laufmittel: n-Heptan/Methylethylketon 2:1, $R_f$ = 0,34

cb) 2-Octadecyloxy-1-naphthol

In einem 10 l-Dreihalskolben mit Rührer, Claisenaufsatz Thermometer und Kühler mit Chlorcalciumrohr gibt man 594 g (1,5 mol) 2-Octadecyloxynaphthalin und 397 g (0,75 mol) Bleitetraacetat in ein Gemisch von 3 l Eisessig und 600 ml Acetanhydrid und erwärmt auf 55 °C. Während 4 Tagen werden im Zeitabstand von 24 Stunden weitere 400 g (jeweils 100 g) Bleitetraacetat portionsweise unter Rühren zugegeben. Danach wird die entstandene gelbe Lösung auf Raumtemperatur abgekühlt, nach Zugabe von 1,5 l Wasser 30 Minuten nachgerührt, der entstandene Kristallbrei abgesaugt und portionsweise mit 2 l Wasser gewaschen. Das feuchte Rohprodukt wird in 4 l Toluol gelöst und dreimal mit je 1 l Wasser, dreimal mit 1 l gesättigter Natriumhydrogencarbonatlösung und dann abermals 3 mal mit 1 l Wasser durchgeschüttelt. Nach Trocknen der Toluolphase über Natriumsulfat, Absaugen und Einengen erhält man 635 g braunes Rohprodukt, das chromatographisch wie folgt gereinigt wird: Man löst das erhaltene Kristallisat in einer Mischung von 1,3 l Toluol/Isohexan 5:2 und gibt die Lösung auf eine Kieselgel 60 (Merck)-säule, innerer Durchmesser 11,5 cm, Füllhöhe 1,2 m. Als Laufmittel verwendet man Toluol/Isohexan 5:2 und schneidet Fraktionen von ca. 300 ml. Die Fraktionen 9-52 werden vereinigt und bis zur Gewichtskonstanz eingeengt. Man erhält 324,2 g 2-Octadecyloxy-1-naphtholacetat, Fp. 67-68 °C. Dieses wird ohne weitere Reinigung in 1,8 l Methanol unter Erwärmen gelöst und auf 20 °C abgekühlt. Zu der entstandenen Suspension tropft man innerhalb 15 Minuten ohne Kühlung unter Rühren 93 ml konz. Schwefelsäure zu, wobei die Temperatur auf 35 °C ansteigt. Anschließend erhitzt man 2 Stunden unter Rückfluß, kühlt dann mit einem Eisbad und rührt 30 Minuten unter Eiskühlung nach. Die gebildeten Kristalle werden abgesaugt, mit 150 ml eiskaltem Methanol gewaschen und bei 35 °C im Trockenschrank über Diphosphorpentoxid getrocknet. Man erhält 294,4 g (47,5 % der Theorie) 2-Octadecyloxy-1-naphthol, farblose Kristalle, Fp. 58-59 °C.

cc) 4-[(2,6- Dibrom-4-nitrophenyl)azo]-2-octadecyloxy-1-naphthol

In einem 2 l Dreihalskolben mit Rührer, Claisenaufsatz und Thermometer werden 22,7 g (0,33 mol) Natriumnitrit während 10-15 Minuten unter Rühren in 300 ml konz. Schwefelsäure eingetragen, wobei man die Temperatur der Reaktionslösung auf 35 °C ansteigen läßt. Man kühlt danach auf 20 °C und tropft in ca. 15-20 Minuten 230 ml Eisessig so zu, daß die Temperatur unter Eiskühlung bei 20-25 °C gehalten wird. Danach gibt man 97,6 ml (0,33 mol) 2,6-Dibrom-4-nitroanilin (Riedel de Haen [99 %GC]) portionsweise während 10 Minuten unter gelegentlichem Kühlen zu, wobei die Temperatur auf19-21 °C gehalten wird und rührt noch 3 Stunden nach. Danach gießt man auf 3,5 l Eiswasser und gibt die entstandene Diazoniumsalzlösung rasch zu einer Lösung von 124 g (0,3 mol) 2-Octadecyloxy-1-naphthol in einer Mischung von 3 l Eisessig und 300 ml Chloroform unter Zugabe von 180 g (1,33 mol) Natriumacetat-trihydrat. (Bei der Herstellung der Lösung des Naphtholethers ist darauf zu achten, daß nach Eintragen desselben in Eisessig-Chloroform unter Zugabe von Natriumacetat nach einem Temperaturanstieg auf ca. 45 °C wieder auf 20 °C abgekühlt wird.) Nach 3 Stunden Rühren im Eisbad wird das erzeugte Kristallisat abgesaugt, der Rückstand dreimal mit je 500 ml Wasser gewaschen und im Trockenschrank bei 40 °C getrocknet. Das Rohprodukt - 295,5 g hellbraune Kristalle - werden chromatographisch gereinigt. Die Azoverbindung wird in 1 l Toluol/Methylenchlorid 2:5 gelöst auf eine Kieselgel 60 (Merck)-säule mit innerem Durchmesser 11,5 cm, Füllhöhe 1,2 m aufgetragen und mit Toluol/Methylenchlorid 2:5 eluiert. Man schneidet Fraktionen von ca. 70 ml. Die Fraktionen 57-173 werden vereinigt und eingeengt. Man erhält 134,2 g braune Kristalle. Diese werden in 480 ml Toluol bei 80 °C gelöst, auf 65 °C gekühlt und unter kräftigem Rühren mit 800 ml Isohexan versetzt. Man läßt unter Rühren auf 20 °C abkühlen, stellt über Nacht in den Kühlschrank, saugt die gebildeten Kristalle ab und wäscht den Filterköcher 2 mal mit 300 ml eiskaltem Toluol/Isohexan 1:1,3 und anschließend mit 300 ml Isohexan. Anschließend trocknet man im Trockenschrank bei 40 °C über Diphosphorpentoxid bis zur Gewichtskonstanz. Man erhält 119,9 g (55,5 % der Theorie) Azoverbindung, hellbraune Kristalle, Fp. 102-103 °C

DC, Kieselgel 60 (Merck), Laufmittel: Toluol-Methylenchlorid 2:5, $R_f$ = 0,38

Transmissionsspektren bei saurem bzw. alkalischem pH in o-Nitrophenyloctylether ergeben $\lambda_{max}$-Werte von 454 bzw. 672 nm.

Zur Beurteilung der Blut-/Plasmadifferenz werden gleiche Testträger zur Untersuchung von Vollblut und von aus Blut durch Zentrifugation wie üblich erhaltenem Plasma eingesetzt. 30 $\mu$l Probe werden auf das Schutznetz (4) pipettiert und der jeweilige Testträger dann in das handelsübliche Reflexionsphotometer Reflotron® (Boehringer Mannheim GmbH, Mannheim, Bundesrepublik Deutschland) eingeführt. Im Reflexionsphotometer wird die Reagenzschicht auf der Klappe mit der Flüssigkeit in der Transportschicht in Berührung gebracht und die ablaufende Reaktion reflexionsphotometrisch gemessen.

Die Ergebnisse sind aus Fig. 2 a und 2 b ersichtlich. Während bei Einsatz eines Glasfaservlieses des Standes der Technik eine merkliche Blut-/Plasmadifferenz zu beobachten ist, sind bei Verwendung erfindungsgemäßer Plasmaabtrennschichten Testträger sowohl für die Analytbestimmung aus Blut als auch aus Plasma geeignet.

**Patentansprüche**

1. Vorrichtung zum hämolysearmen Abtrennen von Plasma aus Vollblut mit einer faserhaltigen Schicht, die eine Erythrozyten aggregierende Substanz enthält dadurch gekennzeichnet, daß die faserhaltige Schicht Glasfasern enthält, die mit Polyvinylalkohol oder Polyvinylalkohol / Polyvinylacetat überzogen sind.

2. Vorrichtung gemaß Anspruch 1, dadurch gekennzeichnet, daß als Erythrozyten aggregierende Substanz kationisches Polymer, Lectin, Antikörper oder eine Mischung einzelner oder aller dieser Substanzen eingesetzt wird.

3. Verfahren zur hämolysearmen Abtrennung von Plasma aus Vollblut, bei dem Vollblut mit einer faserhaltigen Schicht, die eine Erythrozyten aggregierende Substanz enthält, kontaktiert wird und Erythrozyten durch diese faserhaltige Schicht zurückgehalten werden, dadurch gekennzeichnet, daß als faserhaltige Schicht eine glasfaserhaltige Schicht eingesetzt wird, deren Glasfasern mit Polyvinylalkohol oder Polyvinylalkohol / Polyvinylacetat überzogen sind.

4. Verfahren gemäß Anspruch 3, dadurch gekennzeichnet, daß als Erythrozyten aggregierende Substanz kationisches Polymer, Lectin, Antikörper oder einer Mischung einzelner oder aller dieser Substanzen eingesetzt wird.

5. Testträger zur Bestimmung eines Blutbestandteiles enthaltend einen Plasmagewinnungs- und einen Nachweisbereich, dadurch gekennzeichnet, daß der Plasmagewinnungsbereich eine Vorrichtung gemäß einem der Ansprüche 1 oder 2 enthält.

6. Verwendung von mit Polyvinylalkohol oder Polyvinylalkohol / Polyvinylacetat überzogenen Glasfasern, die eine Erythrozyten aggregierende Substanz tragen zur hämolysearmen Abtrennung von Plasma aus Vollblut.

7. Verwendung einer glasfaserhaltigen Schicht, in der die Glasfasern mit Polyvinylalkohol oder Polyvinylalkohol / Polyvinylacetat überzogen sind zur Herstellung einer Vorrichtung gemäß Anspruch 1 oder 2.

8. Verwendung einer glasfaserhaltigen Schicht gemäß Anspruch 7, wobei die Schicht eine Erythrozyten aggregierende Substanz enthält.

**Claims**

1. Device for the low-haemolysis separation of plasma from whole blood with a fibre-containing layer which contains a substance aggregating erythrocytes, characterised in that the fibre-containing layer contains glass fibres which are coated with polyvinyl alcohol or polyvinyl alcohol/polyvinyl acetate.

2. Device according to claim 1, characterised in that, as erythrocyte-aggregating substance, there is used cationic polymer, lectin, antibody or a mixture of individual or of all of these substances.

3. Process for the low-haemolysis separation of plasma from whole blood in which whole blood is contacted with a fibre-containing layer which contains an erythrocyte-aggregating substance and erythrocytes are retained by this fibre-containing layer, characterised in that, as fibre-containing layer, there is used a glass fibre-containing layer, the glass fibres of which are coated with polyvinyl alcohol or polyvinyl alcohol/polyvinyl acetate.

4. Process according to claim 3, characterised in that, as erythrocyte-aggregating substance, there is used cationic polymer, lectin, antibody or a mixture of individual or of all of these substances.

5. Test carrier for the determination of a blood component containing a plasma-obtaining and a detection region, characterised in that the plasma-obtaining region contains a device according to one of claims 1 or 2.

6. Use of glass fibres coated with polyvinyl alcohol or polyvinyl alcohol/polyvinyl acetate which carry an erythrocyte-aggregating substance for the low-haemolysis separation of plasma from whole blood.

7. Use of a glass fibre-containing layer in which the glass fibres are coated with polyvinyl alcohol or polyvinyl alcohol/polyvinyl acetate for the production of a device according to claim 1 or 2.

8. Use of a glass fibre-containing layer according to claim 7, whereby the layer contains an erythrocyte-aggregating substance.

**Revendications**

1. Dispositif pour la séparation, avec seulement une faible hémolyse, du plasma à partir du sang total, comportant une couche fibreuse qui contient une substance provoquant l'aggrégation des érythrocytes, caractérisé en ce que la couche fibreuse contient des fibres de verre qui sont revêtues de poly(alcool vinylique) ou de poly(alcool vinylique) / poly(acétate de vinyle).

2. Dispositif selon la revendication 1, caractérisé en ce que, comme substance provoquant l'aggrégation des érythrocytes, on utilise un polymère cationique, de la lectine, un anticorps ou un mélange de quelques-unes ou de la totalité de ces substances.

3. Procédé pour la séparation, avec seulement une faible hémolyse, du plasma à partir du sang total, dans lequel le sang total est mis en contact avec une couche fibreuse qui contient une substance provoquant l'aggrégation des érythrocytes, et les érythrocytes sont retenus par cette couche fibreuse, caractérisé en ce que, comme couche fibreuse, on utilise une couche contenant des fibres de verre, où les fibres de verre sont revêtues de poly(alcool vinylique) ou de poly(alcool vinylique) / poly(acétate de vinyle).

4. Procédé selon la revendication 3, caractérisé en ce que, comme substance provoquant l'aggrégation des érythrocytes, on utilise un polymère cationique, de la lectine, un anticorps ou un mélange de quelques-unes ou de la totalité de ces substances.

5. Support de test pour la détermination d'un constituant du sang, contenant une zone d'obtention du plasma et une zone de détection, caractérisé en ce que la zone d'obtention du plasma contient un dispositif selon l'une des revendications 1 ou 2.

6. Utilisation de fibres de verre revêtues de poly(alcool vinylique) ou de poly(alcool vinylique) / poly-(acétate de vinyle), qui portent une substance provoquant l'aggrégation des érythrocytes pour la séparation, ave seulement une faible hémolyse, du plasma à partir du sang total.

7. Utilisation d'une couche fibreuse, dans laquelle les fibres de verre sont revêtues de poly(alcool vinylique) ou de poly(alcool vinylique) / poly(acétate de vinyle), pour la préparation d'une dispositif selon la revendication 1 ou 2.

8. Utilisation d'une couche fibreuse selon la revendication 7, dans laquelle la couche contient une substance provoquant l'aggrégation des érythrocytes.

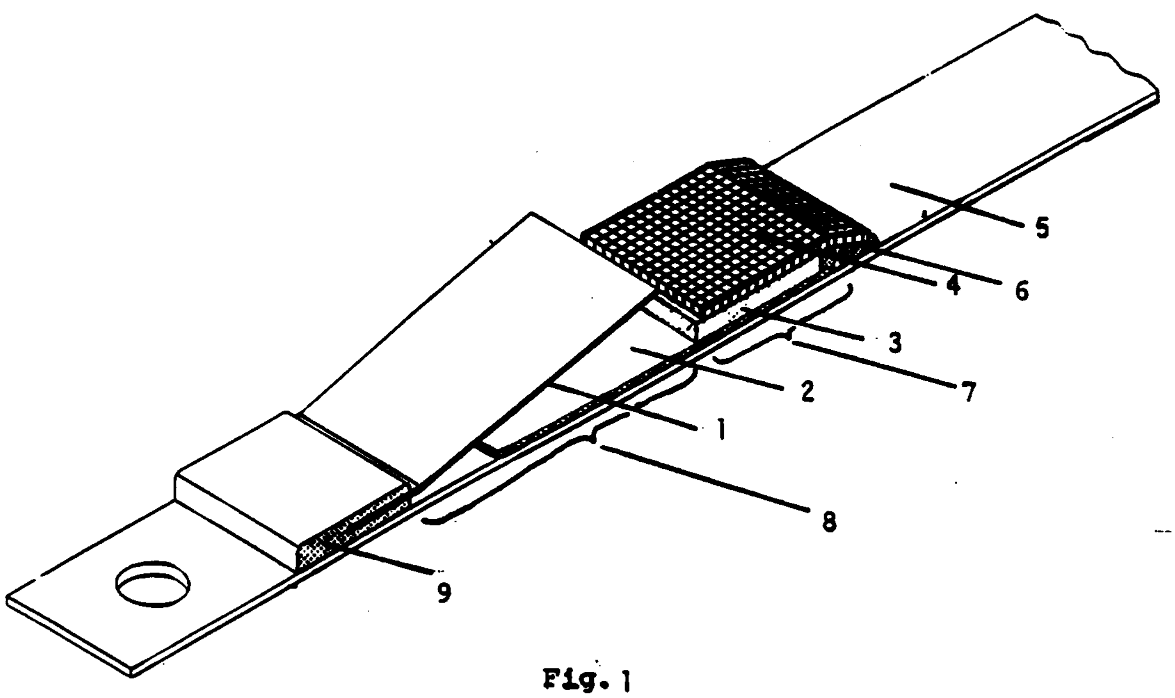


Fig. 1

Fig. 2 a

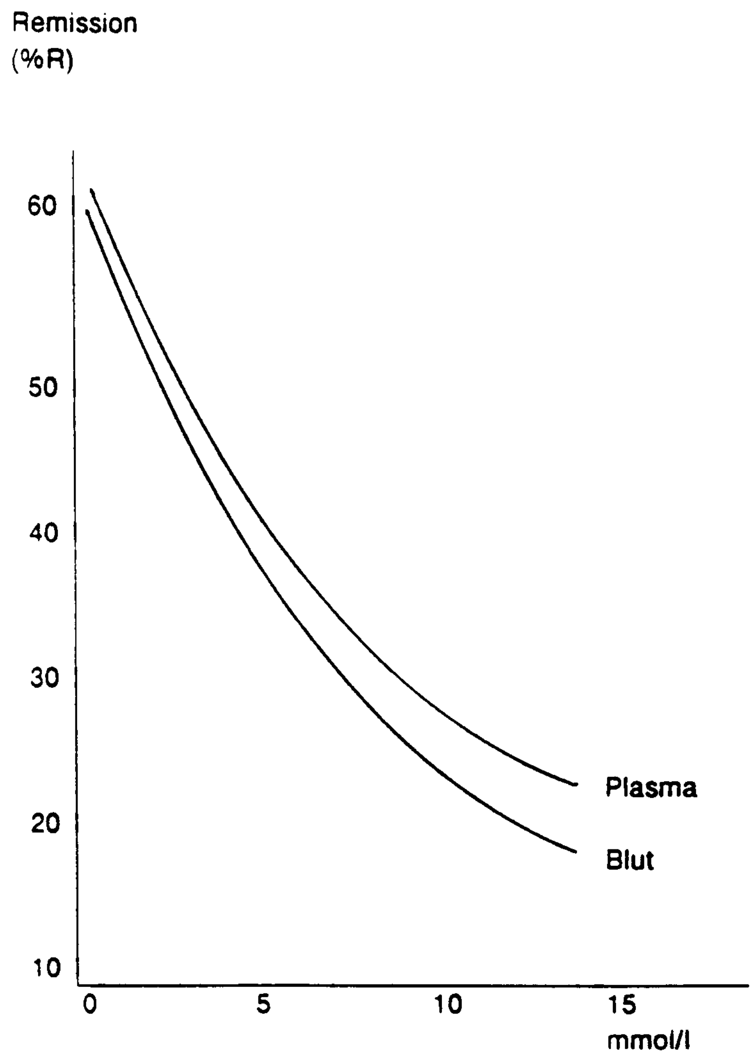

Fig. 2 b